# EUROPEAN PATENT APPLICATION

(11) **EP 1 685 898 A1**
(43) Date of publication of application: **02.08.2006**
(21) Application number: 04799557.6
(22) Date of filing: 02.11.2004
(51) Int. Cl.: B01J 31/36, C07C 11/107, C07C 2/30, C07C 2/22

(54) **TRIMERIZATION CATALYST FOR OLEFIN**

(30) Priority: 06.11.2003 JP 2003376589
(71) Applicant: Sumitomo Chemical Company, Limited, Tokyo 104-8260 (JP)
(72) Inventor: YANAGAWA, Masao, Akashi-shi Hyogo 6730003 (JP); IWAKURA, Kazunori, Toyonaka-shi Osaka 5600026 (JP); ODA, Seiji, Ibaraki-shi Osaka 5670841 (JP)
(74) Representative: Vossius & Partner
(86) International application number: PCT/JP2004/016619
(87) International publication number: WO 2005/044445

(57) **Abstract**

The present invention provides a catalyst comprising
(A) a tantalum compound, and
(B) an organic metal compound, wherein the organic metal compound (B) comprises at least one group selected from the group consisting of the following (1) to (5):
   (1) a branched or cycloalkyl-substituted primary alkyl group having 4 to 15 carbon atoms,
   (2) an aryl-substituted primary alkyl group having 7 to 15 carbon atoms,
   (3) a 3-alkenyl group having 4 to 15 carbon atoms,
   (4) a secondary alkyl group having 3 to 15 carbon atoms which may be substituted with an aryl group or a cyclic alkyl group having 3 to 15 carbon atoms, and
   (5) a secondary alkenyl group having 4 to 15 carbon atoms, the catalyst showing good olefin trimerizing activity.

## Description

### Technical Field

The present invention relates to a trimerization catalyst for olefin, and a process for trimerizing an olefin using the catalyst.

### Background Art

As a process of using a tantalum compound to selectively trimerize an olefin, inparticular, ethylene, there are disclosed in USP No. 6344594andJ. Am. Chem. Soc. , 2001, 123, 7423acatalyst composition comprising a tantalum compound and an alkylating agent. As the alkylating agent, disclosed are tetramethyltin, tetraethyltin, allyltriphenyltin, tetra-n-butyltin, tetraphenyltin, dimethylzinc, diethylzinc, trimethylaluminum, triethylaluminum, methylaluminoxane, dimethylaluminum chloride, methyllithium, n-butyllithium, tert-butyllithium, phenyllithium, methylmagnesium bromide, and the like. However, the ethylene-trimerizing process using the catalyst composition disclosed in the above-mentioned documents has a problem in that the catalytic activity thereof is not necessarily sufficient.

### Disclosure of the Invention

According to the invention, a catalyst having a good catalytic activity can be obtained, and an olefin can be effectively trimerized by use of this catalyst.

Accordingly, the invention provides:
an olefin-trimerizing catalyst comprising
   (A) a tantalum compound, and
   (B) an organic metal compound, wherein the organic metal compound (B) comprises at least one group selected from the group consisting of the following (1) to (5):
      (1) a branched or cycloalkyl-substituted primary alkyl group having 4 to 15 carbon atoms,
      (2) an aryl-substituted primary alkyl group having 7 to 15 carbon atoms,
      (3) a 3-alkenyl group having 4 to 15 carbon atoms,
      (4) a secondary alkyl group having 3 to 15 carbon atoms which may be substituted with an aryl group or a cyclic alkyl group having 3 to 15 carbon atoms, and
      (5) a secondary alkenyl group having 4 to 15 carbon atoms ; and a process for trimerizing an olefin in the presence of the above-mentioned olefin-trimerizing catalyst.

### Best Mode for Carrying Out the Invention

The tantalum compound (A) in the invention include a tantalum halide, an alkoxide tantalum and the like. Preferred is a tantalum halide , and is more preferably tantalum (V) fluoride, tantalum (V) chloride, tantalum (V) bromide, tantalum (V) iodide, and the like.

A description will be made to the groups (1) to (5) which constitute the organic metal compound (B) used in the invention are described.
(1) Specific examples of the branched or cycloalkyl-substituted primary alkyl group having 4 to 15 carbon atoms include isobutyl, 2-methylbutyl, 2-ethylbutyl, 2,2-dimethylpropyl, isopentyl, 2-methylpentyl, isohexyl, 2-methylhexyl, 2-ethylhexyl, cyclopentylmethyl, and cyclohexylmethyl groups. Preferred are the isobutyl, 2-methylbutyl, 2-methylpentyl, cyclopentylmethyl, and cyclohexylmethyl groups. More preferred is the isobutyl group.
(2) Specific examples of the aryl-substituted primary alkyl group having 7 to 15 carbon atoms include benzyl, 2-phenylethyl, and 2-phenylpropyl groups. Preferred is the 2-phenylethyl group.
(3) Specific examples of the 3-alkenyl group having 4 to 15 carbon atoms(homo-allyl type group) include 3-butenyl, 2-methyl-3-butenyl, and 3-methyl-3-butenyl groups. Preferred are primary or secondary 3-alkenyl groups having 4 to 15 carbon atoms, a specific example thereof being the 3-butenyl group.
(4) Specific examples of the a secondary alkyl group having 3 to 15 carbon atoms which may be substituted with an aryl group or a cyclic alkyl group having 3 to 15 carbon atoms include isopropyl, sec-butyl, 2-(3-methyl)-butyl, 2-pentyl, cyclopentyl, cyclohexyl, and 1-phenylethyl groups. Preferred are the isopropyl, sec-butyl, cyclopropyl, cyclopentyl, cyclohexyl, and 1-phenylethyl groups.
(5) Specific examples of the secondary alkenyl group having 4 to 15 carbon atoms include 1-methyl-2-propenyl, 3-(1-pentenyl), 3-(4-methyl-1-pentenyl), and 1,2-dimethyl-2-propenyl groups. Preferred is the 1-methyl-2-propenyl group.

Of the above, the following are more preferred as the organic metal compound (B) comprising at least one group selected from the group consisting of isopropyl, isobutyl, sec-butyl, 3-butenyl, cyclopentylmethyl, cyclohexylmethyl, 1-phenylethyl, and 2-phenylethyl groups. Most preferred is a compound comprising isobutyl group.

The organic metal compound (B) in the invention includes, as examples thereof, the following alkylating agents.

Specific examples of the organic metal compound (B) include, for example, alkyllithium, alkylmagnesium halides, alkylaluminum, alkylaluminoxane, and alkyltin.

Specific examples of the alkyllithium include isopropyllithium, sec-butyllithium, isobutyllithium, cyclopentyllithium, 2,2-dimethylpropyllithium, cyclohexyllithium, 1-phenylethyllithium, and 2-phenylethyllithium. Preferred are isopropyllithium, sec-butyllithium, isobutyllithium and the like.

Specific examples of the alkylmagnesium halides include isopropylmagnesium halides, cyclopentylmagnesium halides, 2,2-dimethylpropylmagnesium halides, cyclohexylhalides, 1-methyl-2-propenylmagnesium halides, 2-phenylethylmagnesium halides, 3-butenylmagnesium halides, cyclopropylmagnesium halides, sec-butylmagnesium halides, isobutylmagnesium halides, 2-ethylhexylmagnesium halides, and 2-ethylbutylmagnesium halides. Preferred are isopropylmagnesium halides, cyclopentylmagnesium halides, cyclohexylhalides, 2-phenylethylmagnesium halides, 3-butenylmagnesium halides, sec-butylmagnesium halides, isobutylmagnesium halides and the like. Preferred are the isopropylmagnesium halides, sec-butylmagnesium halides, and isobutylmagnesium halides. Examples of the halides in the alkylmagnesium halides above include chlorine, bromine, iodine and the like.

Specific examples of the alkylaluminum include triisopropylaluminum, triisobuytlaluminum, tri-sec-butylaluminum, tricyclopentylaluminum, tri(2,2-dimethylpropyl)aluminum, tricyclohexylaluminum, diisopropylaluminum chloride, isopropylaluminum dichloride, diisobutylaluminum chloride, isobutylaluminum dichloride, di-sec-butylaluminum chloride, sec-butylaluminum dichloride, and diisobutylaluminum hydride. Preferred are triisopropylaluminum, triisobuytlaluminum, tri-sec-butylaluminum, diisobutylaluminum chloride, isobutylaluminum dichloride, and diisobutylaluminum hydride. More preferred is triisobutylaluminum.

Examples of the alkylaluminoxane include cyclic aluminoxane having a structure represented by the following formula (1):

{-Al(R¹)ₐ(R²)₁₋ₐ-O-}_{b} (1)

wherein R¹ represents a hydrogen atom or a hydrocarbon having 1 to 8 carbon atoms, R² represents a primary alkyl group which has a branch and has 4 to 15 carbon atoms, a primary alkyl group which has an aryl group as a substituent and has 7 to 15 carbon atoms, a homo-allyl group which has 4 to 15 carbon atoms, a secondary alkyl group which has 3 to 15 carbon atoms, or a secondary alkenyl group which has 4 to 15 carbon atoms, a represents a numerical value of 0 or more and less than 1, and b represents an integer of 2 or more; or linear aluminoxane having a structure represented by the formula (2):

R³{-Al(R¹)ₐ(R²)₁₋ₐ-O-}_{c}Al(R⁴)(R⁵) (2)

wherein R¹, R² and a have the same meanings as described above, R³, R⁴ and R⁵, which may be the same or different, each represents a hydrogen atom, or a hydrocarbon group having 1 to 8 carbon atoms, and c represents an integer of 1 or more.

Specific examples of the hydrocarbon having 1 to 8 carbon atoms for R¹, R³, R⁴ or R⁵ include methyl, ethyl, n-propyl, isopropyl, n-butyl, sec-butyl, isobutyl and tert-butyl groups. Preferred are the methyl, and isobutyl groups.

In R², specific examples of the primary alkyl group which has a branch and has 4 to 15 carbon atoms, the primary alkyl group which has an aryl group as a substituent and has 7 to 15 carbon atoms, the homo-allyl group which has 4 to 15 carbon atoms, the secondary alkyl group which has 3 to 15 carbon atoms, or the secondary alkenyl group which has 4 to 15 carbon atoms, include the substituents described above. Preferred is isobutyl group. "a" represents a numerical value of 0 or more and less than 1. "b" is an integer of 2 or more, and "c" represents an integer of 1 or more. Preferably, b is an integer of 2 to 40 and c is an integer of 1 to 40.

Specific examples of the aluminoxane include a modified methylaluminoxane (aluminoxane wherein methyl groups in methylaluminoxane are partially substituted with isobutyl groups), and isobutylaluminoxane.

The above-mentioned aluminoxane can be produced by various methods, which are not particularly limited. The aluminoxane can be produced in accordance with a known method. For example, the aluminoxane can be produced by bringing water into contact with a solution wherein a trialkylaluminum (such as triisobutylaluminum) is dissolved in a suitable organic solvent (such as benzene or an aliphatic hydrocarbon). Furthermore, an example of the known method is a method of bringing a trialkylaluminum (such as triisobutylaluminum) into contact with a metal salt containing crystal water (such as copper sulfate hydrate). A commercially available product may be used. Specific examples of the alkyltin include tetraisopropyltin, isopropyltrimethyltin, tetraisobutyltin, tetra(2,2-dimethylpropyl)tin, diisobutyltin dichloride, tetra-sec-butyltin, and tetracyclohexyltin. Preferred are tetraisopropyltin, tetraisobutyltin, tetra-sec-butyltin, and the like.

The above-mentioned alkylating agents may be used alone or in the form of a mixture of two or more thereof.

The olefin-trimerizing catalyst of the invention, which comprises a tantalum compound and an alkylating agent, can be prepared by bringing the tantalum compound and the alkylating agent into contact with each other. The method for the preparation is not particularly limited. For example, the catalyst can be prepared by bringing the tantalum compound and the alkylating agent into contact with each other in a solvent, or by contacting them with each other without using any solvent, and then adding a solvent to the resultant. The contact of the tantalum compound with the alkylating agent is beforehand conducted, and subsequently the mixture is brought into contact with an olefin, whereby the trimerization can be carried out. The contact of the tantalum compound with the alkylating agent is conducted in the presence of an olefin, whereby the trimerizing reaction can be started at the same time when the tantalum compound is brought into contact with the alkylating agent. The mixing order of these starting materials is not particularly limited. Preferably, the alkylating agent is added to the tantalum compound.

In the preparation of the catalyst of the invention and the trimerizing reaction, a solvent is used. Examples of the solvent used herein include aliphatic hydrocarbons such as butane, pentane, hexane, heptane, octane, isooctane, cyclopentane, methylcyclopentane, cyclohexane, methylcyclohexane, cyclooctane, and decalin; aromatic hydrocarbons such as benzene, toluene, xylene, cumene, ethylbenzene, monochlorobenzene, or dichlorobenzene; and halogenated hydrocarbons such as dichloromethane, chloroform, carbon tetrachloride, or dichloroethane. The olefin itself which is the starting material for the trimerizing reaction, or a reaction product olefin such as 1-butene, hexene or octene, can be used as the solvent.

The solvent is preferably an aromatic hydrocarbon, and is more preferably benzene, toluene, xylene, monochlorobenzene, dichlorobenzene or the like. These solvents may be used alone or in the form of a mixture of two or more thereof.

The concentration of the tantalum compound when the catalyst of the invention is prepared is not particularly limited, and is usually from 0.0001 µmol to 100 mmol, preferably from 0.001 µmol to 10 mmol.

In the catalyst of the invention, the amount of the alkylating agent is usually from 0.5 to 4 times by mole, preferably from 0.7 to 3 moles, more preferably from 1 to 2.5 moles in terms of the alkyl group(s) per mol of the tantalum compound. The wording "in terms of the alkyl group(s)" means that a number corresponding to the mole number of the alkyl group(s) which can react with the tantalum compound, and can be represented as the product of the mole number of the alkylating agent per mol of the tantalum compound and the number of the alkyl group(s) that the alkylating agent has. For example, when the amount of isopropyllithium is 1 mole per mol of the tantalum compound, the amount in terms of the alkyl group(s)is 1 mole. When the amount of diisopropylzinc is 1 mole per mol of the compound, the amount in terms of the alkyl group(s) is 2 moles.

The temperature when the catalyst of the invention is prepared is usually from -100 to 250°C, preferably from -78 to 150°C. The time for the preparation is not particularly limited, and is usually from 0 minute to 24 hours.

The preparation of the catalyst of the invention and the trimerizing reaction are preferably conducted in an inert gas atmosphere while the contact with water is avoided. Thus, it is preferred that the compounds to be used are sufficiently dried in advance.

The thus-prepared catalyst is used to carry out the trimerizing reaction of an olefin. The amount of the catalyst of the invention is not particularly limited, and is usually from 0.001 µmol to 100 mmol, preferably from 0.01 µmol to 10 mmol per liter of the solvent.

Examples of the olefin which is used as the starting material in the invention include α-olefins such as ethylene, propylene, 1-butene, 1-pentene, 1-hexene, 1-heptene, 1-octene, 1-nonene, or 1-decene; internal olefins such as 2-butene, 2-pentene, 2-hexene, 3-hexene, 2-octene, 3-octene, and 4-octene; branched olefins such as isobutylene, 3-methyl-1-butene, 4-methyl-1-pentene, or 2-ethyl-1-hexene; and diolefins such as 1,3-butadiene, isoprene, 1,4-pentadiene, or 1,5-hexadiene.

The catalyst for the present reaction is highly active and particularly suitable for the trimerizing reaction of ethylene, and makes it possible to yield 1-hexene with a high activity and a high selectivity.

The temperature of the trimerizing reaction in the invention is usually from -100 to 250°C, preferably 150°C or lower, more preferably from 0 to 200°C, even more preferably from 10 to 80°C. The reaction pressure is usually an absolute pressure of from normal pressure to a pressurized pressure, preferably of from normal pressure to 300 MPa, more preferably of from normal pressure to 30 MPa. The reaction time is not particularly limited, and is usually from 0 minute to 24 hours.

The starting material olefin may be continuously supplied to keep the above-mentioned pressure, or may be sealed up at the above-mentioned pressure as the reaction is started. The olefin diluted with nitrogen, argon, helium or the like may be used.

The present reaction can be carried out in a batch-wise manner, semi-continuous manner, or continuous manner. After the end of the reaction, an inactivating agent such as water, an alcohol, hydrochloric acid, or an aqueous sodium hydroxide solution, is added to the reaction solution, thereby terminating the reaction. After completion of the reaction, the inactivated catalyst is removed by a known demineralization treatment such as extraction with water or an aqueous alkaline solution. Thereafter, the desired olefin can be separated by a known operation such as distillation or extraction.

The trimerizing reaction of an olefin is preferably carried out under a reaction condition wherein the water content in the reaction system is 50 moles or less per mole of the tantalum atom and the amount of molecular oxygen in the reaction system is 10 moles or less per mole of the tantalum atom.

### Examples

The present invention will be described in more detail by way of the following examples. However, the invention is not limited to these examples.

### Example 1

Toluene was charged into an autoclave in the atmosphere of ethylene, the pressure of which was normal pressure, so as to set the total amount thereof to 5 mL, and thereto was added 0.8 mL of a solution of 22.4 mg of tantalum pentachloride dissolved in 10 mL of toluene (the amount of tantalum pentachloride: 5 µmol). The temperature thereof was then stabilized at 40°C. Thereafter, the pressure of ethylene was increased to 0.6 MPa, and stabilized. Thereinto was charged 13 µL (3.3 µmol) of triisobutylaluminum (manufactured by Tosoh Fine Chemicals Pte Ltd., 0.25 mol/L solution in toluene), and ethylene was caused to react for a time shown in Table 1. The reaction container was cooled to room temperature, and next the pressure was returned to normal pressure. The reaction solution was analyzed by gas chromatography. A solid matter contained in the reaction solution was filtrated off with filter paper. This solid was air-dried and then dried at a reduced pressure. The weight thereof was then measured. The results are shown in Table 1.

### Example 2

The reaction operations were carried out in a similar manner as in Example 1 except using the kind of an alkylating agent, a charged amount thereof and a reaction time shown in Table 1. The results are shown in Table 1. PBAO represents polyisobutylaluminoxane.

| Examples | 1 | 2 |
|---|---|---|
| TaCl5: µmol | 5.0 | 5.0 |
| Alkylating agent µmol | TIBA 3.3 | PBAO 10.0 |
| Reaction time: hour(s) | 0.44 | 1.5 |
| Reaction temperature: °C | 40 | 40 |
| | | |
| Components: wt% | | |
| C4 | 10.3 (99.0) | 3.4 (91.2) |
| C6 | 84.2 (99.0) | 95.4 (98.7) |
| C8 | 0.9 (0) | 0.0 |
| C10 | 3.7 (0) | 1.2 (0) |
| C12 | 0.0 | 0.0 |
| C14 | 0.2 (0) | 0.0 |
| C16 | 0.0 | 0.0 |
| Solid component (PE) | 0.7 | 0.0 |
| Activity: mol/(mol(cat.)-hr) | | |
| 1-Butene | 190 | 9 |
| 1-Hexene | 1034 | 176 |
| 1-Octene | 0 | 0 |
| 1-Decene | 0 | 0 |
| 1-Dodecene | 0 | 0 |
| 1-Tetradecene | 0 | 0 |
| 1-Hexadecene | 0 | 0 |

| | | |
|---|---|---|
| C4: Butene, C6: Hexene, C8: Octene, C10: Decene, C12: Dodecene, | | |
| C14: Tetradecene, C16: Hexadecene | | |

The number in parentheses in each column for the components represents the purity of linear α-olefins in the each of the components ((the amount of linear α-olefins/the total amount of isomers of each of the components) ×100)
TIBA: Triisobutylaluminum
PBAO: Polyisobutylaluminoxane (manufactured by Tosoh Fine Chemicals Pte Ltd.)

### Example 3

Toluene was charged into an autoclave in the atmosphere of ethylene, the pressure of which was normal pressure, so as to set the total amount thereof to 5 mL, and thereto was added 0.8 mL of a solution of 167.9 mg of tantalum pentachloride suspended in 15 mL of toluene (the amount of tantalum pentachloride: 25 µmol). The temperature thereof was then stabilized at 40°C. Thereafter, the pressure of ethylene was increased to 0.6 MPa, and stabilized. Thereinto was charged 26 µL (50 µmol) of MMAO-3A (modified methylaluminoxane manufactured by Tosoh Fine Chemicals Pte Ltd., 1.9 mol/L solution in toluene), and ethylene was caused to react for 0.2 hour. The reaction container was cooled to room temperature, and next the pressure was returned to normal pressure. The reaction solution was analyzed by gas chromatography. A solid matter contained in the reaction solution was filtrated off with filter paper. This solid was air-dried and then dried at a reduced pressure. The weight thereof was then measured. The results are shown in Table 2.

### Comparative Example 1

The same operations as in Example 3 were carried out except that dimethylzinc was used instead of MMAO-3A and the reaction was conducted for a reaction time shown in Table 2. The results are shown in Table 2.

**Table 2**

| | Example 3 | Comparative Example 1 |
|---|---|---|
| TaC15: µmol | 25 | 25 |
| Alkylating agent µmol | MMAO-3A 50 | ZnMe2 25 |
| Reaction time: hour(s) | 0.2 | 1.3 |
| Reaction temperature: °C | 40 | 40 |
| | | |
| Components: wt% | | |
| C4 | 3.3 (100) | 12.3 (98.4) |
| C6 | 87.4 (97.4) | 76.4 (86.9) |
| C8 | 3.1 (3.2) | 1.9 (0) |
| C10 | 6.2 (0) | 3.4 (0) |
| C12 | 0.6 (16.7) | 0.2 (0) |
| C14 | 1.2 (0) | 0.0 |
| C16 | 0.0 | 0.0 |
| Solid component (PE) | 1.4 | 0.0 |
| Activity: mol/(mol(cat.)-hr) | | |
| 1-Butene | 33 | 12 |
| 1-Hexene | 568 | 42 |
| 1-Octene | 0 | 0 |
| 1-Decene | 0 | 0 |
| 1-Dodecene | 0 | 0 |
| 1-Tetradecene | 0 | 0 |
| 1-Hexadecene | 0 | 0 |

| | | |
|---|---|---|
| C4: Butene, C6: Hexene, C8: Octene, C10: Decene, C12: Dodecene, | | |
| C14: Tetradecene, C16: Hexadecene | | |

The number in parentheses in each column for the components represents the purity of linear α-olefins in the each of the components ((the amount of linear α-olefins/the total amount of isomers of each of the components) × 100)
MMAO-3A: Modifiedmethylaluminoxane (manufactured by Tosoh Fine Chemicals Pte Ltd.)

### Example 4

Toluene was charged into an autoclave in the atmosphere of ethylene, the pressure of which was normal pressure, so as to set the total amount thereof to 5 mL, and thereto was added 125 µL of a solution of 100.3 mg of tantalum pentachloride suspended in 14 mL of toluene (the amount of tantalum pentachloride: 2.5 µmol). The temperature thereof was then stabilized at 70°C. Thereafter, the pressure of ethylene was increased to 0.6 MPa, and stabilized. Thereinto was charged 67 µL (1.7 µmol) of triisobutylaluminum (TIBA manufactured by Tosoh Fine Chemicals Pte Ltd. , 0.025 mol/L solution in toluene), and ethylene was caused to react for 3 hours. The reaction container was cooled to room temperature, and next the pressure was returned to normal pressure. The reaction solution was analyzed by gas chromatography. A solid content contained in the reaction solution was filtrated off with filter paper. This solid was air-dried and then dried at a reduced pressure. The weight thereof was then measured. The results are shown in Table 3.

### Example 5

The reaction operations were carried out in a similar manner as in Example 4 except that o-dichlorobenzene was used instead of toluene as the solvent in Example 4. The results are shown in Table 3.

### Comparative Example 2

The reaction operations were carried out in a similar manner as in Example 4 except that dimethylzinc was used instead of triisobutylaluminum in Example 4. The results are shown in Table 3.

**Table 3**

| Examples | 4 | 5 | Comparative Example 2 |
|---|---|---|---|
| TaCl5: µmol | 2.5 | 2.5 | 3 |
| Alkylating agent µmol | TIBA 1.7 | TIBA 1.7 | ZnMe2 3 |
| Reaction time: hour(s) | 3.0 | 3.0 | 3.0 |
| Reaction temperature: °C | 70 | 70 | 70 |
| Solvent | Toluene | o-Dichlorobenzene | Toluene |
| Components: wt% | | | |
| C4 | 1.1 (100) | 1.0 (100) | 0.2 (100) |
| C6 | 94.4 (99.9) | 86.9 (99.8) | 96.5 (100) |
| C8 | 0.1 (0.0) | 0.7 (0.0) | 0.1 (100) |
| C10 | 4.3 (65.1) | 10.8 (50.9) | 3.2 (0) |
| C12 | 0.0 | 0.0 | 0.0 |
| C14 | 0.0 | 0.6 (0.0) | 0.0 |
| C16 | 0.0 | 0.0 | 0.0 |
| Solid component (PE) | 0.0 | 0.0 | 0.0 |
| Activity: mol/(mol(cat.)-hr) | | | |
| 1-Butene | 6 | 8 | 0 |
| 1-Hexene | 356 | 460 | 100 |
| 1-Octene | 0 | 0 | 0 |
| 1-Decene | 3 | 5 | 0 |
| 1-Dodecene | 0 | 0 | 0 |
| 1-Tetradecene | 0 | 0 | 0 |
| 1-Hexadecene | 0 | 0 | 0 |

| | | | |
|---|---|---|---|
| C4: Butene, C6: Hexene, C8: Octene, C10: Decene, C12: Dodecene, | | | |
| C14: Tetradecene, C16: Hexadecene | | | |

The number in parentheses in each column for the components represents the purity of linear α-olefins in the each of the components ((the amount of linear α-olefins/the total amount of isomers of each of the components) ×100)

### Industrial Applicability

According to the present invention, a catalyst having a good catalytic activity can be obtained, and an olefin can be trimerized by use of this catalyst.

## Claims

1. A catalyst comprising
(A) a tantalum compound, and
(B) an organic metal compound, wherein the organic metal compound (B) comprises at least one group selected from the group consisting of the following (1) to (5):
(1) a branched or cycloalkyl-substituted primary alkyl group having 4 to 15 carbon atoms,
(2) an aryl-substituted primary alkyl group having 7 to 15 carbon atoms,
(3) a 3-alkenyl group having 4 to 15 carbon atoms,
(4) a secondary alkyl group having 3 to 15 carbon atoms which may be substituted with an aryl group or a cyclic alkyl group having 3 to 15 carbon atoms, and
(5) a secondary alkenyl group having 4 to 15 carbon atoms.

2. The catalyst according to claim 1, wherein the tantalum compound (A) is a tantalum halide.

3. The catalyst according to claim 1 or 2, wherein the organic metal compound (B) comprises at least one group selected from the group consisting of isopropyl, isobutyl, sec-butyl, homo-allyl, cyclopentylmethyl, cyclohexylmethyl, 1-phenethyl, and 2-phenethyl groups.

4. The catalyst according to claim 1 or 2, wherein the organic metal compound (B) comprises isobutyl group.

5. The catalyst according to claim 1 or 2, wherein the organic metal compound (B) is an isopropylmagnesium halide, an isobutylmagnesium halide, a sec-butylmagnesium halide, a cyclopentylmagnesium halide, a cyclohexylmagnesium halide, a 1-phenethylmagnesium halide, a 2-phenethylmagnesium halide, isopropyllithium, isobutyllithium, sec-butyllithium, cyclopentyllithium, cyclohexyllithium, 1-phenethyllithium, 2-phenethyllithium, triisopropylaluminum, triisobutylaluminum, tri-sec-butylaluminum, tricyclohexylaluminum, isobutylaluminum dichloride, diisobutylaluminum chloride, a diisobutylaluminum halide, a modified methylaluminoxane, isobutylaluminoxane, tetraisopropyltin, isopropyltrimethyltin, tetraisobutyltin or a diisobutyltin dihalide.

6. The catalyst according to claim 1 or 2, wherein the organic metal compound (B) is triisobutylaluminum, a modified methylaluminoxane, or isobutylaluminoxane.

7. The catalyst according to any one of claims 1 to 6, wherein the amount of the organic metal compound (B) is from 0.5 to 3 moles in terms of the alkyl group(s) per mole of the tantalum compound (A).

8. The catalyst according to any one of claims 1 to 7, wherein the olefin is ethylene.

9. The catalyst according to any one of claims 1 to 8, which is obtained by contacting the tantalum compound (A) with the organic metal compound (B).

10. An olefin-trimerizing process, which comprises trimerizing an olefin in the presence of the catalyst according to any one of claims 1 to 9.

11. The olefin-trimerizing process according to claim 10, which is carried out at an absolute pressure of from normal pressure to a pressurized pressure.

12. The olefin-trimerizing process according to claim 11, wherein the absolute pressure is from normal pressure to 30 MPa.

13. The olefin-trimerizing process according to any one of claims 10 to 12, which is carried out at a temperature of 150°C or lower.

14. The olefin-trimerizing process according to claim 13, which is carried out at a temperature of 10 to 80°C.

15. The olefin-trimerizing process according to any one of claims 10 to 14, which is carried out in the presence of a solvent.

16. The olefin-trimerizing process according to claim 15, wherein the solvent is an aromatic compound.

17. The olefin-trimerizing process according to claim 15, wherein the solvent is at least one selected from the group consisting of benzene, toluene, xylene, chlorobenzene and dichlorobenzene.

18. The olefin-trimerizing process according to any one of claims 10 to 17, wherein the olefin is ethylene.
